# EUROPEAN PATENT SPECIFICATION

(11) **EP 0 642 302 B1**
(45) Date of publication and mention of the grant of the patent: **12.08.1998**
(21) Application number: 93908558.5
(22) Date of filing: 24.03.1993
(51) Int. Cl.: A01N 33/04, A01N 33/10, C07C 217/10, C07C 323/26

(54) **METHOD FOR ENHANCING PLANT GROWTH BY APPLYING A COMPOUND, AND SUITABLE COMPOUNDS**
VERFAHREN ZUR STEIGERUNG DES PFLANZENWACHSTUMS DURCH VERABREICHUNG EINER VERBINDUNG UND GEEIGNETE VERBINDUNGEN
METHODE POUR ACCELERER LA CROISSANCES DES PLANTES PAR APPLICATION D'UN COMPOSE E COMPOSES APPROPRIES

(30) Priority: 30.03.1992 US 860413; 30.09.1992 US 954725; 30.09.1992 US 954726
(43) Date of publication of application: 15.03.1995
(73) Proprietor: TROPICANA PRODUCTS, INC., Bradenton, FL 34206 (US); THE UNITED STATES OF AMERICA as represented by THE SECRETARY OF AGRICULTURE, Washington, DC 20231 (US)
(72) Inventor: YOKOYAMA, Henry, Pasadena, CA 91105 (US); KEITHLY, James, H., Bradenton, Florida 34208 (US); GAUSMAN, Harold, W., Amarillo, TX 79109 (US)
(74) Representative: Kupecz, Arpad
(86) International application number: PCT/US93/02770
(87) International publication number: WO 93/19597

(56) References cited:
- DE-A- 1 926 755
- GB-A- 1 224 676
- GB-A- 1 239 567
- US-A- 2 766 238
- US-A- 2 769 839
- US-A- 3 558 640
- US-A- 3 833 350
- US-A- 4 797 153
- Bio/Technology, March 1985, GAUSMAN et al., "Effect of 2 Diethylamino ethyl-3,4- Dichlorophenylether (DCPTA) on Cotton Plant (Gossypium Hirsutum) Growth and Phenology", (no page numbers given), see entire article.
- Plant Growth Regulation 9, 1990, KEITHLY et al., "Regulation of Plant Productivity I: Improved Seeding Vigor and Floral Performance of Phalaenopsis by 2-(3.4-dichlorophenoxy)triethylamine (DCPTA)", pp. 19-26.

## Description

### Field of Invention

The present invention is directed to a method of enhancing plant growth by applying a compound to a plant, and enhancing properties associated with plants that have been treated with the compound, and preferred chemical compounds capable of enhancing plant growth. Application of the compounds results in an increase in sugar content, essential oils, proteins and an increase in total plant biomass. Fruits harvested from treated plants exhibit an accelerated biochemical and structural maturity. Mature fruits typically exhibit increased pigment accumulation, increased essential oil accumulation, and reduced peel thickness. The methods include application of individual bioregulator compounds, mixtures thereof, and in vitro application of individual compounds and mixtures thereof. Certain mixtures of the bioregulator compounds exhibit synergistic effects. The new compounds function as plant bioregulators and thus enhance plant growth in accordance with the method of the invention.

### Background of the Invention

Developments in agriculture have produced chemical compounds and methods for their application which function as plant bioregulators and thus serve to enhance one or more properties exhibited by the treated plant. For example, United States Patent Number 3,671,219 discloses a quartenary ammonium compound which when applied to plants enhances the sugar content of sugar cane. United States Patent Number 4,204,859 discloses that the addition of certain phenoxytrialkylamines enhance the hydrocarbon production of rubber in plants. United States Patent Number 4,159,903 discloses a method for increasing polyisoprene production in rubber producing plants such as Guayule. United States Patent Number 3,833,350 discloses that carotenoid accumulation in plants can be increased according to a method comprised of applying compounds including (halogenated phenoxy) trialkylamines. United States Patent Numbers 3,864,501, 3,911,148, and 3,911,152 disclose a method for increasing the carotenoid pigments of fruits and vegetables which comprises the application of compounds including (methyl phenoxy) trialkylamines.

United States Patent Numbers 3,558,640, 2,766,238 and United Kingdom Patent Number 1,239,567 disclose compounds related to those according to the invention but for medical purposes.

United States Patent Number 4,797,153 discloses a method for increasing total plant biomass and individual plant constituents such as sugar, protein, lipid, and essential oils wherein certain substituted phenoxytrialkylamines and substituted phenylthiotrialkyl amines, or dialkylmorpholinium halides are applied to plants. The compounds are applied in bioregulatory amounts to plant seeds, plant seedlings, or plant buds at the early stage of plant development, or to trees a week before or after flower bud swell. It has since been shown that the application of these compounds in bioregulatory amounts facilitates the assimilation of carbon dioxide in the photosynthetic pathway of green plants, thereby increasing the carbon atoms available for synthesis of total biomass and individual plant constituents.

### Summary of the Invention

The present invention is directed towards a method of enhancing plant growth by applying (benzyl substituted) trialkylamine ether compounds (either individually or in mixtures) to plants in regulatory amounts resulting in increased important plant constituents, increased total plant biomass, and increased rate of plant growth, and reduced time to crop maturity. Pigment accumulation in plant leaves and mature fruits is increased. In Citrus crops, the fruits harvested from treated trees exhibit a reduced peel thickness. The compounds are applied to the plants in bioregulatory amounts - that is, an amount sufficient to increase plant biomass and accelerate growth but insufficient to harm the plant. The compounds to be applied according to the method of the present invention are selected from the group of chemical compounds having the structure: 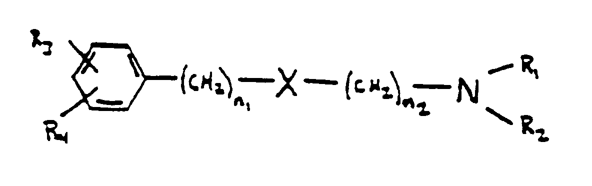
wherein X is either oxygen or sulfur,
R₁ and R₂ are lower alkyl groups containing 1 to 6 carbon atoms each of identical or dissimilar structure,
n₁ and n₂ are integers from 1 to 6, with n₁ and n₂ being independent of each other,
R₃ and R₄ are independently hydrogen, chlorine, bromine, fluorine, iodine lower alkyl compounds containing 1 to 6 carbon atoms, lower alkoxy containing 1 to 6 carbon atoms, and wherein:
if R₃ and R₄ are 3,5-substituents, then the lower alkyl or alkoxy group must contain 3 to 6 carbon atoms; and wherein: if R₃ is hydrogen, then R₄ must be a 4-substituent, with the proviso that R₄ is other than hydrogen; or
b) an acid addition salt of the compounds defined above.

It has been found that the application of these compounds or mixtures thereof causes the treated plants to form and store valuable plant constituents over untreated plants. The plants which have been treated with the bioregulatory compounds of the invention have greater biomass than untreated plants resulting in increased crop production per unit area.

Moreover, field studies have been conducted in which the compounds have been compared to the bioregulator compounds disclosed in United States Patent Number 4,797,153, specifically 3,4-dichlorophenoxy triethylamine (3,4-DCPTA) and 2,4-dichlorophenoxytriethylamine (2,4-DCPTA). It was determined that a compound of the present invention known as N,N-diethylaminoethyl (4-methylbenzyl) ether (MBTA) is generally more effective as a plant bioregulator than the bioregulator compounds disclosed in the '153 patent. That is, MBTA treated plants exhibit a greater increase in total plant biomass and valuable plant constituents relative to DCPTA. A second compound of the invention, N,N-diethylaminoethyl 3,4-dichlorobenzyl ether (DCBTA) performs comparably as a bioregulator with respect to the DCPTA. Thus, the compounds to be applied according to the method of the present invention exhibit a structure-activity correlation at least comparable or even superior to the disclosed prior art and thus represent an advance in the state of the art of bioregulator applications.

Moreover, plants treated with mixtures of the compounds exhibit enhanced metabolic activity in forming and storing valuable plant constituents and increased plant-biomass when compared to plants treated with individual bioregulator agents, which is an unpredicted and unexpected result. Mixtures of the compounds exhibit a greater than additive effect when they are combined as bioregulatory agents and yield synergistic results relative to plants treated with individual bioregulator agents.

We have further discovered that application of a mixture of DCBTA and MBTA to plants effects an unpredicted and unexpected enhancement of plant metabolic activity in forming and storing valuable plant constituents and in increasing plant biomass with respect to similar treatments of individual bioregulator agents, including the DCPTA disclosed in United States Patent No. 4,797,153 and the MBTA and DCBTA disclosed herein. The mixture of MBTA and DCBTA, when applied to plants, results in a greater than additive bioregulatory effect when compared to treatments of the aforementioned individual bioregulator agents. This greater than additive effect, or synergistic effect, further advances the state of the art.

In many cases the invention increases the growth rate of the treated plant relative to untreated plants, resulting in accelerated maturation. Moreover, accelerated and increased growth make likely the possibility that growing cycles will be shorter while yielding a harvest equivalent or superior to that of untreated plants. Such a harvest would be greater since the treated plants exhibit increased biomass.

The present invention is also directed to a method of in vitro application of the bioregulator compounds and mixtures thereof. One particular aspect of in vitro applications concerns the usage of compounds and mixtures as growth inducing agents for orchid seed germination and germinated orchid seed (protocorm). The addition of the bioregulator compounds to an aseptic culture medium significantly enhances orchid protocorm growth and substantially reduces the period of time for which they are cultured to produce mature, blooming plants.

### Detailed Description of the Preferred Embodiment

The benefits of the invention are obtained by applying any of the following compounds, or mixtures thereof, to leaves, to plant seeds, seedling plant buds, immature fruits, or vegetative propagules. "Mixtures" as used herein, refers to a combination of at least any two compounds encompassed by claim 1. Examples, by way of illustration and not limitation, of compounds that can be used in the process of the invention are:
A. N,N-dialkylaminoalkyl 2,4-substituted benzyl ethers wherein the 2,4-substituents are independently chloro, bromo, iodo, methyl, ethyl, propyl, butyl, pentyl, hexyl, methoxy, ethoxy, propoxy, butoxy, pentoxy or hexoxy, and wherein the alkyl groups are independently either methyl, ethyl, propyl, butyl or pentyl or isomers thereof.
B. N,N-dialkylaminoalkyl 3,5-substituted benzyl ethers wherein the 3,5-substituents are independently chloro, bromo, iodo, propyl, butyl, pentyl, hexyl, propoxy, butoxy, pentoxy or hexoxy, and wherein the alkyl groups are the same as those in A.
C. N,N-dialkylaminoalkyl 3,4-substituted benzyl ethers wherein the 3,4-substituents are independently chloro, bromo, iodo, methyl, ethyl, propyl, butyl, pentyl, hexyl, methoxy, ethoxy, proproxy, butoxy, pentoxy or hexoxy, and wherein the alkyl groups are the same as those in A.
D. N,N-dialkylaminoalkyl 4-substituted benzyl ether wherein the 4-substituent is either methyl, ethyl, propyl, butyl, pentyl, hexyl, methoxy, ethoxy, propoxy, butoxy, pentoxy or hexoxy, and wherein the alkyl groups are the same as those in A.

The preferred compounds of the present invention as set forth in groups A through D are those where n₁ is 1 and n₂ is 2, X is oxygen, the N-alkyl groups are both ethyl, and the benzyl substituents are 2,4-dichloro; 3,4-dichloro; 3-5-diisopropyl; 3,5,-ditertiary butyl; 3,4-dimethyl; 3,4-dimethoxy; 3-methyl, 4-methyl or 4-chloro.

It has been found that two particular compounds are especially preferred in that plants treated with either of these compounds exhibit significant improvements in total plant biomass and individual plant constituents, and in particular compare favorably to the bioregulator compounds disclosed in the United States Patent 4,797,153. These compounds are N,N-diethylaminoethyl 3,4-dichlorobenzyl ether (DCBTA) and N,N-diethylaminoethyl 4-methylbenzyl ether (MBTA). It has been found that a mixture of these two compounds is also preferred in that plants treated with this mixture exhibit significant improvements in total plant biomass and individual plant constituents with respect to plants treated with individual bioregulator compounds. Mixtures are preferably comprised of equal amounts (1:1, w/w) of each compound.

Various acid addition salts of the above compounds can be readily produced. For example, by adding an acid, the following acid addition salts are formed:

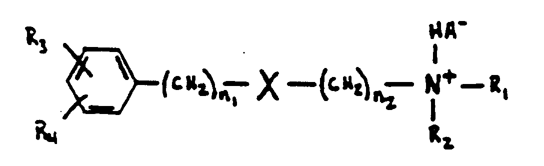

Wherein the molecular constituents are as set forth above, and wherein A is the anion derived from the acid added to the amine to form a salt. Mixtures of acid addition salts can also be used as bioregulatory agents.

In order to achieve increase in total biomass yield, enhancement of individual plant constituents or increase in rate of plant growth, the compounds or mixtures thereof must be first applied to the plant at an early stage of development That is, immediately prior to, or at the time when cell differentiation and plant growth are great. If application is made at a late stage of development, some increase in yield or plant constituents may occur but not the significant increase which occurs when treatment is earlier. As a practical matter, treatment is made to the seed such as cereal grain; to the post-emergent seedling plant, that is, to the plant at or prior to the full expansion of the fourth set of primary leaves, such as at the cotyledon, true leaf, two-leaf or four-leaf stage; or to trees during flower bud swell or a week before or after. For plants which are not grown from seed or do not produce flower buds such as vegetatively propagated plants like sugarcane, application should be at the developmental growth stages equivalent to the ones mentioned above. Since growth of the plant or tree dilutes the concentration of the bioregulatory application due to increase in plant biomass resulting in a biomass dilution effect, it may be desirable to apply more than one application subsequent to the initial one. Subsequent applications should be made before completion of cell differentiation of the growing plant or when applied to a growing tree before the completion of cell differentiation of the growing fruit.

Generally, where the compounds or mixtures thereof are applied to the seeds, the concentration is 0.001 to 0.3 mg of active ingredient per seed. Application is conveniently made by dissolving the compound to be used in water at a concentration of 0.1 to 50 parts per million (ppm) in the diluent and soaking the seeds for 2 to 6 hours. Other means of treatment of seeds such as encapsulation of the seeds with the compounds by conventional methods are encompassed by the invention.

When compounds or mixtures thereof are applied to the seedling, that is at the cotyledon, true leaf, two-leaf or four-leaf stages and the like, the treatment is 0.001 mg to 0.3 mg active ingredient per plant. This can be accomplished by using a treatment rate of 0.1 to 200 ppm and preferably 5 to 120 ppm. Use of treatment rates of 300 ppm or greater on young seedlings or young plants, in other words, prior to the full expansion of the fourth set of primary leaves, will either not cause increases in biomass contemplated by the invention or in many cases, may have a phytotoxic effect on the plant causing it to have stunted growth.

Treatment of perennial trees requires a greater amount of the bioregulator compound or mixture due to the greater mass of the tree. Generally, one to four grams active ingredient per tree is applied using a treatment rate of 0.1 to 500 ppm of bioregulatory compound.

The compounds or mixtures may be applied to the plant in any convenient manner. For example, after being dissolved in water, the compound or mixture can be sprayed onto the branches and leaves of the plant. Other application techniques known to the skilled artisan may be employed.

Appropriate wetting agents such as Triton X-100™ (polyethylene glycol p-isooctylphenylether made by J. T. Baker), ORTHO X-77™ (a mixture of fatty acids, fatty alcohols and isopropanol made by Chevron Chemical Company), Sweep 4F™ (chlorothalonil from Diamond Shamrock Company) and the like may be added to the aqueous solution to aid in plant treatment. Appropriate penetrating agents such as B-cyclodextrin (B-(heptamer)-cyclodextrin made by Takeda Chemical Industries, Ltd.) or Tween 80™ (polyoxyethylene (20) sorbitan monooleate, available from E. Merck, Darmstadt Germany) may be added to the aqueous solution to increase penetration of the bioregulatory compound. Solutions of bioregulator and appropriate wetting agent may be adjusted to an acidic pH (pH 4 to 5) prior to plant application.

With respect to pH, the applicant has found the following conditions to be preferable. When ungerminated seeds are hydrated in solutions of pH 5 to 7, seedling growth was optimized (solutions contained 10 ppm MBTA 0.1% Tween 80™(%).

Seedling root growth was significantly enhanced by MBTA within test solutions of pH 3 to 6.

Hypocotyl growth was enhanced by MBTA within test solutions of pH 4 to 5.

The results suggest that incubation of seeds in pH 3 to 5 solutions may facilitate the transport of protonated bioregulator into plant tissues.

When the compounds or mixtures thereof are used in vitro, any number of isotonic, buffered, nutrient media containing mineral salts as macronutrients and micronutrients, hormones, vitamins and supplements may be utilized in accordance with the present invention so long as they are capable of supporting propagation of the subject plant. For example germination of orchid seeds and orchid protocorm on Hill's seed germination medium, available from Gallup and Sterling Laboratories, Santa Barbara, CA., was shown to be acceptable.

In order to achieve beneficial results with in vitro applications, the compounds or mixtures of compounds should be introduced into the nutrient media at the earliest stages of plant development. Most preferably, this will be after sterilization of the medium and before or contemporaneous with seed or propagule sowing. It should be understood that application of the bioregulators is best made prior to solidification of the agar support. If application is made later some increase in yield or plant constituents may occur but not the significant increase which occurs if treatment is earlier. Since the growth of the plant or propagule dilutes the concentration of the chemical mixture due to increase in plant biomass resulting in a biomass dilution effect, it may be desirable to apply more than one application subsequent to the initial one. Subsequent applications should be made before completion of cell differentiation of the growing plant or when applied to a growing propagule before the completion of cell differentiation of the growing fruit.

Generally, where the mixtures are applied to the seeds in vitro, application is conveniently made by dissolving the compounds or mixture to be used in water at a concentration of 0.1 to 50 parts per billion (ppb) and introducing the compounds to the sterilized culture medium. It is preferred that if a mixture is used then the mixture can be comprised of equal amounts of each bioregulator agent in the mixture.

Treatment of perennial trees propagated in vitro requires a greater amount of the bioregulator mixture due to the greater mass of the tree. Generally, 0.01 to 10 mg total active ingredients per tree is applied using a treatment rate of 1 to 100 ppb of bioregulatory mixtures. However, we have found that bioregulatory effects result from applications as low as 0.01 ppb.

When applied in accordance with the method of the invention the compounds or mixtures of the invention substantially increase total biomass, enhance the amount of some or all plant constituents and in many cases increase the rate of growth in green plants over untreated plants as long as constituents such as water and light necessary for plant growth are present in the required amount.

Using the method of invention, seed treatment of radish resulted in a greatly enhanced root and leaf development at crop harvest as compared with controls. Seed or foliar treatment of petunia, verbena, aster, and other ornamental crops increased root development, secondary branching and increased bud count per plant. Treated ornamental crops typically flower sooner and have a greatly improved aesthetic appeal. Treatment of Citrus trees causes the fruit to mature faster, to bear an increased fruit yield, to increase the Vitamin C content and to produce fruits with an increased essential oil content. USDA color score values of juice recovered from treated Citrus is superior due to an increased pigment content. Thus, the method of the invention finds use on any green plant where increased rate of growth, biomass or the like is desired. The method is particularly valuable for use on plants which produce food, vitamins, nutrients, fiber, or energy; or on plants where commercial production is limited due to low plant yield when grown without bioregulators. The composition and method can be used on annual or perennial plants, such as seasonal row crops, vineyards, orchards, and all ornamental or horticultural plants.

For example, treated Hamlin, Valencia, and Pineapple sweet orange trees show an accelerated ripening of fruit. When compared with untreated controls, treatment of Citrus trees produces mature fruits that have an increased brix, essential oil, and vitamin C contents. Juice color is enhanced by chemical treatment. Thus, the method of the invention produces significant improvements in the nutritional and sensory qualities of Citrus products and reduces the time to harvest of mature Citrus fruits. Similar reductions in the days to crop maturation have been observed in a variety of ornamental crop plants (aster, verbena, petunia, pansy).

### EXAMPLE 1

Two year old grafted trees of 'Okitsu-wase' Satsuma mandarin as well as two year old "Kara" and "Kinnow" grafted trees were planted into 25 liter pots and were maintained under 40% saran cover in Pasadena, California. The following tertiary amines were prepared as 100 ppm solutions (pH 5.0) in 0.5% Tween 80™ (v/v):
N,N-diethylaminoethyl 3,4-dichlorophenylether (DCPTA) (a/k/a (3,4-dichlorophenoxy) trialkylamine)
N,N-diethylaminoethyl 3,4-dichlorobenzylether (DCBTA)

The bioregulator solutions were applied to foliage runoff in a single application. Each treatment group contained two trees. Control trees were sprayed to foliage runoff using 0.5% Tween 80™ (v/v). At the time of bioregulator application, Satsuma fruit diameters ranged from 0.8 to 1.4 cm. During fruit development, the trees were fertilized every 14 days using a 20N-20P-20K soluble fertilizer and the trees received a monthly side-dressing of Ironite. Mature Satsuma fruits were harvested months after bioregulator application. In each bioregulator treatment group, mature fruits from the two replicate trees were combined for fruit quality analysis. For analysis, five fruits of approximately 50 to 55 mm in diameter were chosen from each treatment group. Total fruit fresh weight was determined. Fruits were cut in half and peel thickness was determined. Fruits were juiced by hand. The combined juice and pulp were pressed through a 0.5 mm sieve and the final juice volume and juice fresh weight were determined. Peel fresh weight after juicing and pulp fresh weight were determined.

**Table 1**

| Variety | Chemical Treatment | ml Juice per g fruit fresh wt | Vitamin C (mg/100ml) | Serum Brix | Peel Thickness (mm) |
|---|---|---|---|---|---|
| Kara | Control | 0.34 b | 20.6 c | 12.9 b | 4.3 b |
| | DCPTA 100 ppm | 0.38 ab | 25.2 b | 14.6 a | 3.3 a |
| | DCBTA 100 ppm | 0.42 a | 29.9 a | 14.4 a | 3.5 a |
| Kinnow | Control | 0.30 b | ND | 15.2 b | 4.7 b |
| | DCPTA 100 ppm | 0.37 a | ND | 15.3 b | 4.1 a |
| | DCBTA 100 ppm | 0.36 a | ND | 15.7 a | 4.2 a |
| Okitsu-wase | Control | 0.27 b | 24.3 b | 11.8 b | 4.6 b |
| | DCPTA 100 ppm | 0.41 a | 25.8 a | 13.1 a | 3.2 a |
| | DCBTA 100 ppm | 0.41 a | 24.8 b | 13.4 a | 3.4 a |
| Letters within columns indicate mean separation according to Duncan's multiple range test, 5% level. ND = not determined. | | | | | |

### EXAMPLE 2

The coordinated improvement of peel structure and juice composition of citrus fruits is measured. Citrus trees were maintained as:
a. Mature orchard trees of 'Olinda' Valencia sweet orange located at the Agricultural Experiment Station, University of California, Riverside, California; and
b. Orchard trees of Hamlin and Pineapple sweet orange located in Arvin, California.

### a. Riverside Field Planting:

Bioregulator treatments occurred in a 24-tree block of 'Olinda' valencia orange. The trees were 10 years old and had been skirted. Each treatment group contained three trees. Chemical treatment groups consisted of: Control; DCPTA-50 ppm; DCPTA-100 ppm; DCBTA-50 ppm; DCBTA-100 ppm; MBTA-50 ppm; MBTA-100 ppm; MBTA-200 ppm. All solutions (pH 5.0) contained 0.5% Tween 80™ (v/v). Bioregulator solutions were applied as a single application. Each tree received 4 liters of bioregulator solution that was applied as evenly as possible to the entire foliage canopy. At the time of bioregulator treatment, fruit sizes ranged from 2 to 3.5 cm in fruit diameter. Fruits were harvested in December, six months after chemical treatment.

**Table 2**

| Chemical Treatment(ppm) | Fruit Diam | ml Juice per g Fruit Fresh wt | Vitamin C (mg/100ml) | Serum ^{.}Brix | Peel mm | % Juice | Fruit Fresh Peel+Pulp | Wt Total |
|---|---|---|---|---|---|---|---|---|
| Control | 65.4 | 0.48 | 43.3 | 9.84 | 4.8 | 50.7 | 47.6 | 98.3 |
| DCPTA-50 | 65.1 | 0.49 | 50.6 | 11.14 | 4.3 | 51.2 | 47.1 | 98.3 |
| DCPTA-100 | 65.2 ns | 0.48 ns | 48.3 L*,Q* | 10.64 L*,Q* | 4.4 L* | 51.1 | 47.5 | 98.6 |
| DCBTA-50 | 65.6 | 0.48 | 47.0 | 10.94 | 4.5 | 51.1 | 47.2 | 98.3 |
| DCBTA-100 | 65.0 ns | 0.49 ns | 47.8* L* | 10.65 L* | 4.6 L* | 50.9 | 47.1 | 98.0 |
| MBTA-50 | 65.2 | 0.49 | 48.2 | 11.44 | 4.2 | 51.6 | 46.9 | 98.5 |
| MBTA-100 | 64.7 | 0.47 | 47.3 | 11.14 | 4.5 | 50.9 | 47.2 | 98.1 |
| MBTA-200 | 64.8 ns | 0.46 ns | 52.0 L* | 10.84 Q* | 4.7 Q* | 50.4 | 48.3 | 98.7 |
| ns, *, L, Q Not significant or significant at P=0.05(*) according to linear (L) or quadratic (Q) models | | | | | | | | |

Fruit diameters and ml fruit/fresh weight remain comparable among controls and all treatment groups (Table 2). Chemical treatments show a reduction in peel thickness relative to controls, while fruit diameters are comparable for all groups including controls. MBTA-50 treated citrus exhibit a significant increase in Brix. When compared with control juice samples, chemical treatment significantly increased the Vitamin C content of Valencia sweet orange. Among all treatments, MBTA-200 ppm treatment resulted in the largest numerical increase in Vitamin C accumulation in mature fruits (Table 2).

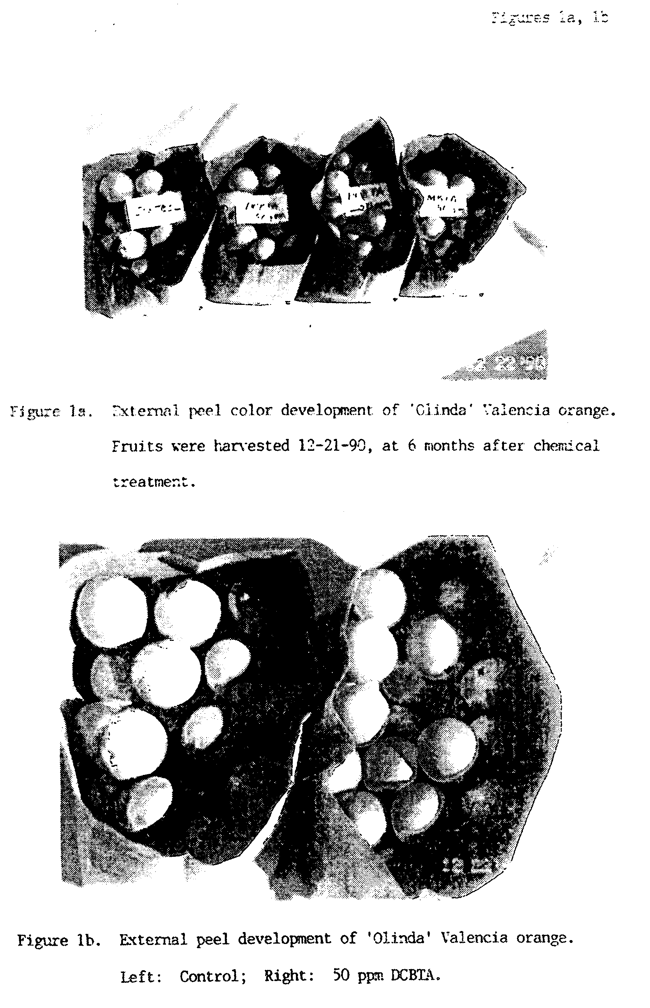

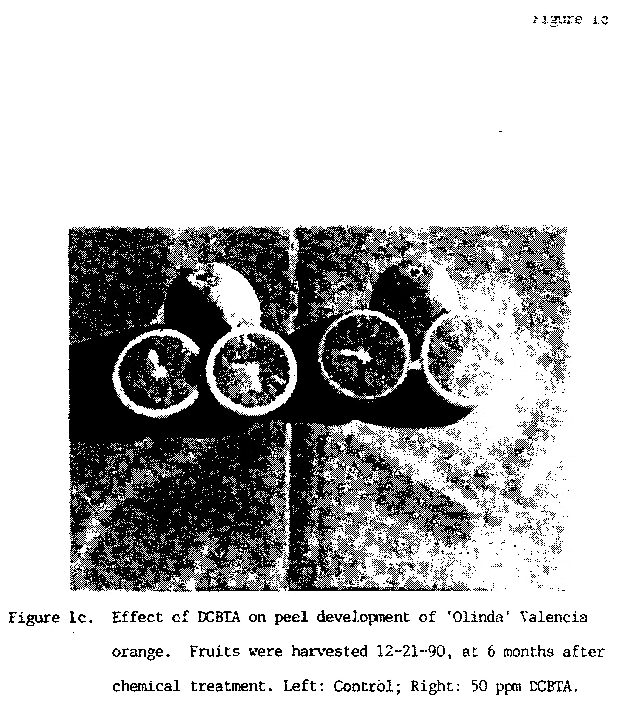

Bioregulator application to fruiting 'Olinda' valencia trees significantly enhanced the flavedo carotenoid accumulation of fruits that were harvested 6 months after chemical treatment (Figures 1a, 1b, 1c). Fruits harvested from 50 ppm DCPTA, 50 ppm DCBTA, and 50 ppm MBTA-treated trees generally showed the most uniform improvements in flavedo carotenoid development (Figure 1a) when compared with control fruits. Sectioned fruits (Figure 1c) from control and bioregulator-treated trees visually showed similar endocarp carotenoid development. However, fruits harvested from bioregulator-treated 'Olinda' trees showed significant reductions in peel thickness (Figure 1c and Table 2).

### b. San Joaquin Valley Field Trials:

Bioregulator treatments were applied to commercial orchard stock of Pineapple, Hamlin, and Valencia sweet orange. Each bioregulator treatment represents one tree for each cultivar. The trees were 12 to 15 years old and have not been skirted. Experimental trees are internal plantings within a 500 tree block. Chemical treatments were applied as a single foliar application and consisted of: Control; DCPTA-100ppm; DCBTA-100; MBTA-100ppm. All solutions (pH 5.0) contained 0.1% Tween 80™ (v/v). Approximately 5 liters of solution were applied to each tree and the foliage canopy was covered as evenly as possible. At the time of chemical treatment, fruit sizes ranged from 1.5 to 3 cm in diameter. Mature fruits of Hamlin and Pineapple orange were harvested as part of a December harvest 6 months after chemical treatment. Fruits within all treatment groups had attained uniform peel color. Medium-sized, canopy fruits were chosen for harvest. The data below represents eight fruits/sample.

**Table 3**

| Chemical Treatment | Fruit Diam | ml Juice per g Fruit fresh wt | Vitamin C (mg/100ml) | Serum ^{.}Brix | Peel mm | % Juice | Fruit Fresh Peel+Pulp | Wt Total |
|---|---|---|---|---|---|---|---|---|
| HAMLIN | | | | | | | | |
| Control | 67.9a | 0.51ab | 44.8c | 11.44c | 5.1b | 52.3 | 45.3 | 97.6 |
| DCPTA-100 | 68.2a | 0.51ab | 52.5b | 11.68bc | 4.7ab | 52.4 | 46.1 | 98.5 |
| DCBTA-100 | 66.6a | 0.54a | 51.2b | 11.74b | 4.5a | 54.1 | 43.6 | 97.7 |
| MBTA-100 | 67.1a | 0.48b | 59.5a | 12.14a | 4.8b | 46.0 | 51.6 | 97.6 |

| PINEAPPLE | | | | | | | | |
|---|---|---|---|---|---|---|---|---|
| Control | 69.0a | 0.51ab | 53.1b | 11.17c | 5.6c | 49.5 | 48.4 | 97.9 |
| DCPTA-100 | 70.8a | 0.54a | 46.3c | 11.37bc | 4.7b | 55.9 | 43.3 | 99.3 |
| DCBTA-100 | 68.8a | 0.55a | 46.1c | 11.97b | 4.3a | 56.2 | 41.3 | 97.5 |
| MBTA-100 | 67.1a | 0.46b | 61.0a | 13.57a | 5.5c | 45.7 | 51.7 | 97.4 |
| Letters within columns indicate mean separations according to Duncan's multiple range test, 5% level. | | | | | | | | |

DCBTA shows a marked increase in juice recovery/fresh fruit weight in Hamlin and Pineapple fruits. For both Hamlin and Pineapple sweet orange, MBTA treatment shows significant improvement in brix and vitamin C content when compared with control fruits. These results indicate that MBTA-treatment resulted in sweeter fruits with an improved nutritional quality.

Application of DCBTA resulted in the best juice recovery in both Hamlin and Pineapple fruits. However, MBTA foliar-application resulted in the largest numerical increase in brix and vitamin C contents when compared with the values of controls. Improved juice recovery was generally related to a reduction in peel thickness (Tables 1, 2, and 3). However, chemical treatment had no significant effect on final fruit size or fruit shape.

### EXAMPLE 3

Four substituted tertiary amines (3,4-DCPTA, 3,4-DCBTA, (2,4-DCBTA), and MBTA) were synthesized and purified according to the methods of Echols, Maier, Poling, and Sterling, 1981, New bioregulators of Gibberellin Biosynthesis in Gibberella Fujikuroi, Phytochemistry 20:433-437; Poling, Hsu, Yokoyama, 1977 Structure Activity Relationships of Chemical Inducers of Carotenoid Biosynthesis, Phytochemistry 14:1933, respectively.

Radish seeds (Raphanus sativus L. cv. Scarlet turnip white tipped) were supplied by Ferry Morse Seed Co., Modesto CA. Seeds were soaked for 6 hrs at 22°C in 0.1, 1.0, 10.0, 50.0, and 100.0 ppm bioregulator solutions. All bioregulator solutions (pH 5.0) contained 0.1% Tween 80™. Control seed lots were soaked for 6 hrs at 22°C in 0.1% Tween 80™. Seeds were planted immediately after chemical treatment. All plants were greenhouse grown using a standardized radish as described previously. Keithly, J.H., H. Kobayashi, H. Yokoyama, and H.W. Gausman 1991 Promotive Effects of Tertiary Amine Bioregulators on Radish (Raphanus sativus) Growth and Development. PGRSA Quarterly 19(3): 182-187.

The growth enhancing properties of the newly synthesized tertiary amine analogs showed significant differences in a standardized radish growth test (Table 4). The growth of nontreated controls and of DCPTA-treated plants were used as reference plant growth systems. The growth of DCBTA-treated plants were numerically and statistically similar to the growth of DCPTA-treated plants.

When compared to the taproot growth of DCPTA-treated plants, the order of compound effectiveness appears to be as follows:
MBTA > 3,4-DCBTA = DCPTA >> 2,4-DCBTA

**Table 4**

| Enhanced Leaf and Taproot Growth of Radish by Tertiary-amine Bioregulators | | | | | | |
|---|---|---|---|---|---|---|
| Bioregulator Abbreviation | Conc ppm | Leaf Dry wt g | Leaf Area dm² | Root Dry wt g | Root Diam mm | Root to Shoot Ratio |
| Control | | 1.04 | 2.64 | 0.82 | 22.89 | 0.79 |
| 3,4-DCPTA | 0.1 | 1.18 | 2.72 | 1.10 | 23.40 | 0.86 |
| | 1.0 | 1.58 | 3.64 | 1.29 | 27.59 | 0.82 |
| | 10.0 | 1.38 | 3.19 | 1.32 | 30.09 | 0.96 |
| | 50.0 | 1.21 | 2.79 | 1.17 | 25.32 | 0.97 |
| | 100.0 | 1.00 Q* | 2.31 Q** | 1.06 Q* | 23.82 Q** | 1.06 L* |
| 3,4-DCBTA | 0.1 | 1.22 | 3.11 | 1.02 | 22.54 | 0.84 |
| | 1.0 | 1.25 | 3.15 | 0.97 | 21.44 | 0.78 |
| | 10.0 | 1.48 | 3.89 | 1.33 | 29.38 | 0.90 |
| | 50.0 | 1.19 | 3.09 | 1.02 | 22.71 | 0.86 |
| | 100.0 | 1.20 Q* | 3.11 Q** | 1.09 Q* | 23.13 Q* | 0.91 NS |
| 2,4-DCBTA | 1.0 | 1.37 | ND^{z} | 1.04 | ND | 0.76 |
| | 10.0 | 1.39 | ND | 1.08 | ND | 0.78 |
| | 100.0 | 1.28 Q* | ND | 1.12 NS | ND | 0.88 NS |
| MBTA | 0.1 | 1.15 | 2.85 | 1.01 | 22.61 | 0.88 |
| | 1.0 | 1.19 | 2.98 | 1.22 | 26.95 | 1.02 |
| | 10.0 | 1.54 | 3.08 | 1.27 | 27.61 | 0.83 |
| | 50.0 | 1.44 | 3.14 | 1.46 | 29.01 | 1.01 |
| | 100.00 | 1.12 Q** | 2.78 Q** | 0.92 Q* | 22.42 Q* | 0.82 NS |
| NS, Q*, Q**, L* Not significant or significant at P=0.05(*) or P-0.01(**) according to Linear (L) or Quadratic(Q) models. | | | | | | |

| | | | | | | |
|---|---|---|---|---|---|---|
| ^{Z}Not determined | | | | | | |

### EXAMPLE 4

Mesophyll chloroplast development during leaf expansion has been shown to regulate the amount of photosynthate available for vegetative crop growth and reproductive plant development. Fruit set and crop yield are often determined by the amounts of partitioned photosynthate that are available during early fruit growth. The effects of DCPTA, DCBTA, and MBTA on chlorophyll accumulation and Rubisco activity in mature leaves of Valencia, Pineapple, and Hamlin sweet oranges is shown herein.

Chemical treatments were performed when a majority of the trees had started a vegetative growth cycle (growth flush) during April, 1990. Bioregulator solutions (pH 5.0) contained 0.5% Tween 80™. Citrus cultivars were divided into treatment groups that contained three trees per treatment. Foliar applications of bioregulator were performed using a trigger-action hand sprayer. Controls received a foliar application of 0.5% Tween 80™. Solutions were applied to the point of foliage runoff. After chemical treatment, trees were arranged as a completely randomized block.

Leaf growth analysis was performed at 6 to 8 weeks after chemical treatment. Individual leaves were harvested from 3 vegetatively similar branches within each treatment group for leaf morphology analysis. Leaves were numbered basipetally from the first visible leaf at the apical meristem. For each leaf, leaf area (dm²), leaf blade length at the midvein, and leaf fresh weight was determined. Specific leaf weights (SLW, g fresh weight/dm² leaf area) were calculated from leaf fresh weight and leaf area data.

When compared with controls, foliar application of DCPTA, DCBTA, and MBTA to Valencia, Pineapple, and Hamlin sweet orange significantly increased the SLW of mature leaves that were harvested at 6 to 8 weeks after chemical treatment (Table 5). Among the bioregulator treatment groups, SLW was numerically similar. Bioregulator application significantly (P=0.05) increased Chl accumulation in mature orange leaves when compared with that of controls. When compared with controls, bioregulator treatment resulted in generally improved total carotenoid accumulation in mature leaves. Rubisco activity was measured in a wide range of leaf ages, and the most reliable enzyme activities were obtained from leaf numbers 15 to 18 basipetally from the apical meristem. Bioregulator treatment appeared to increase the CCS of sweet orange (Table 6). In all orange cultivars, the soluble protein to Chl ratio was increased significantly (P=0.05) in all chemical treatment groups when compared with controls. Within the chemical treatment groups, the soluble protein to Chl ratios of 50ppm treatments often appeared to be superior to 100ppm treatments. When compared with controls, the observed improvements in leaf soluble protein to Ch1 ratios within the chemical treatment groups supported a significantly (P=0.05) increased Rubisco activity per mg Ch1 (Table 6). Within all treatment groups among all cultivars, 50ppm MBTA application appeared to be one of the most useful chemical treatments. Rubisco activity was not determined in all treatment groups due to the limited amount of experimental material.

The in vitro Rubisco analysis suggests that MBTA may be a very effective chemical regulator of chloroplast development.

**TABLE 5**

| Cultivar | Bioregulator Treatment-PPM | SLW^{z} g/dm² | Pigments mg/dm² | | | |
|---|---|---|---|---|---|---|
| | | | Ch1 a | Ch1 b | Ch1 Total | Car Total |
| Valencia | Control | 2.60c | 4.0d | 1.6d | 5.6c | 1.3d |
| | DCPTA-50 | 3.01ab | 5.4b | 2.7a | 8.1a | 2.0a |
| | DCPTA-100 | 2.95b | 5.1c | 2.3bc | 7.4b | 1.7c |
| | DCBTA-50 | 3.04a | 5.6ab | 2.5b | 8.1a | 1.8b |
| | DCBTA-100 | 3.02ab | 5.5b | 2.5b | 8.0a | 1.8b |
| | MBTA-50 | 2.99b | 5.7a | 2.1c | 7.8ab | 1.8b |
| | MBTA-100 | 3.05a | 5.1c | 2.1c | 7.2b | 1.8b |
| Pineapple | Control | 2.66c | 4.7b | 1.7de | 6.5c | 1.4c |
| | DCPTA-50 | 3.29ab | 4.9b | 2.5b | 7.4b | 1.4c |
| | DCPTA-100 | 3.47a | 4.0c | 1.5e | 5.5d | 1.7a |
| | DCBTA-50 | 3.18b | 5.6a | 2.1c | 7.7a | 1.5b |
| | DCBTA-100 | 2.96b | 5.2ab | 2.0d | 7.2b | 1.4c |
| | MBTA-50 | 3.49a | 4.7a | 2.8a | 7.5a | 1.6a |
| | MBTA-100 | NOT DETERMINED | | | | |
| Hamlin | Control | 2.33d | 3.9d | 1.5c | 5.4e | 1.4d |
| | DCPTA-50 | 2.72bc | 4.9b | 1.8b | 6.7c | 1.7c |
| | DCPTA-100 | 2.65c | 4.6c | 1.8b | 6.4d | 1.6c |
| | DCBTA-50 | 2.81b | NOT DETERMINED | | | |
| | DCBTA-100 | 2.79b | 5.1b | 2.2a | 7.3b | 1.7b |
| | MBTA-50 | 3.04a | 5.2a | 2.5a | 7.7a | 1.8a |
| | MBTA-100 | 2.77b | NOT DETERMINED | | | |

| | | | | | | |
|---|---|---|---|---|---|---|
| ^{Z}Specific leaf weight (g fresh weight/dm²). Determined on leaf numbers 15 to 18 numbered basipetally from the apical meristem. Letters within columns indicate significant differences (cultivars analyzed separately) according to Duncan's multiple range test, 5% level. | | | | | | |

**TABLE 6**

| Enhanced soluble protein accumulation and Rubisco activity of sweet orange leaves by tertiary-amine bioregulators | | | |
|---|---|---|---|
| Treatment | Soluble Protein To Ch1 ratio^{Z} | Total Activated Rubisco Activity | |
| | | Activity/mg protein | Activity/mg Ch1 |
| VALENCIA | | | |
| Control | 14.52d | 2.83a | 41.09d |
| DCPTA-50 | 15.99ab | 2.81a | 44.93b |
| DCPTA-100 | 15.63c | 2.82a | 44.08c |
| DCBTA-50 | 15.95ab | 2.84a | 45.30ab |
| DCBTA-100 | 15.92ab | 2.78a | 44.26c |
| MBTA-50 | 16.24a | 2.84a | 46.12a |
| MBTA-100 | 15.86b | 2.83a | 44.90b |

| PINEAPPLE | | | |
|---|---|---|---|
| Control | 15.65c | 2.83a | 44.29c |
| DCPTA-50 | 16.33a | 2.78a | 45.40a |
| DCPTA-100 | NOT DETERMINED | | |
| DCBTA-50 | 16.21a | 2.77a | 44.90b |
| DCBTA-100 | 15.90b | 2.80a | 44.52c |
| MBTA-50 | 16.19ab | 2.81a | 45.49a |
| MBTA-100 | NOT DETERMINED | | |

| HAMLIN | | | |
|---|---|---|---|
| Control | 14.92c | 2.77a | 41.33d |
| DCPTA-50 | 15.98b | 2.82a | 45.06c |
| DCPTA-100 | 16.01b | 2.79a | 44.67c |
| DCBTA-50 | 16.92a | 2.83a | 47.88a |
| DCBTA-100 | 16.74a | 2.80a | 46.87b |
| MBTA-50 | 16.40ab | 2.83a | 46.41b |
| MBTA-100 | NOT DETERMINED | | |

| | | | |
|---|---|---|---|
| ^{Z}mg protein/(mg Ch1) in Citrus leaf chloroplast preparations ^{Y}Rubisco activity = mg CO₂/h. Letters within columns indicate mean separations according to Duncan's multiple range test, 5% level. | | | |

### EXAMPLE 5

Orchard trees of 'Olinda' Valencia orange were maintained at the Agricultural Experiment Station, University of California, Riverside, California. Twenty four trees were treated with tertiary amine bioregulators as foliage treatments. Each treatment group contained three trees. The randomized complete block experimental design contained the following bioregulator treatments: Control; DCPTA-50 ppm; DCPTA-100 ppm; DCBTA-50 ppm; DCBTA-100 ppm; MBTA-50 ppm; MBTA-100 ppm; and MBTA-200 ppm. All bioregulator solutions (pH 5.0) contained 0.1% Tween 80™ (v/v). Fruits were harvested at 181 days, 215 days, and 259 days after bioregulator treatment (DAT). Sized fruits were analyzed for peel thickness, juice recovery, juice brix, and total peel pigment accumulation. Each fruit sample contained eight randomly chosen fruits.

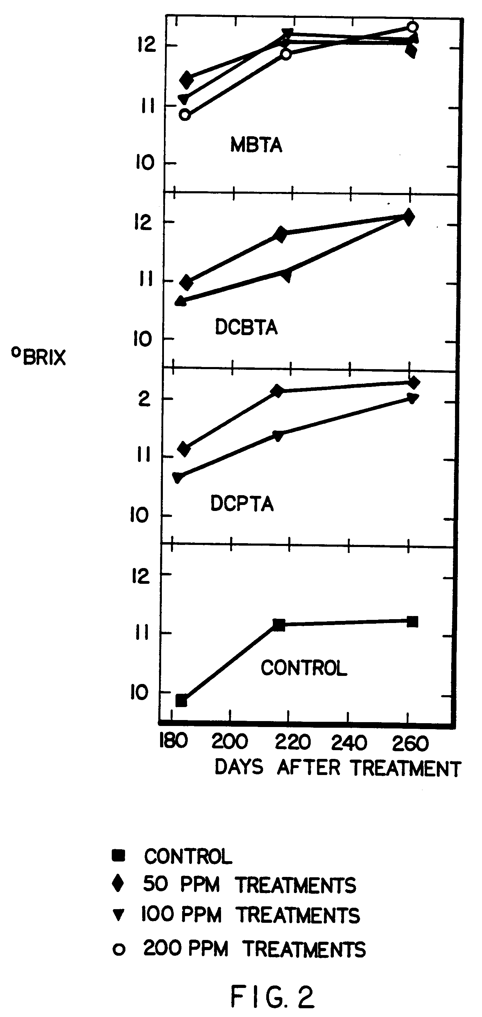

Compared with controls, fruits harvested from bioregulator-treated 'Olinda' trees showed improved peel development and juice accumulation during fruit maturation (Table 7). All fruits harvested from bioregulator-treated trees showed a general reduction in peel thickness as compared with the peel development of controls. Among the three fruit harvests, total soluble solids (brix) accumulation in treated fruits was increased significantly as compared with controls (Figure 2).

Fruits harvested from bioregulator-treated trees showed significant improvements in peel pigment accumulation when compared with controls (Table 8). Among all treatments, chlorophyll content was inversely related to total carotenoid content. The biological activities of DCBTA and MBTA on carotenoid accumulation appeared greater than that of DCPTA.

The brix and carotenoid accumulation of 'Olinda' fruits harvested from bioregulator-treated trees indicates that chemical treatment has reduced the days to fruit harvest by approximately 40 days when compared with controls. Among all treatments, improved juice recovery is related to a reduction in peel thickness. Of the test bioregulators, MBTA appears to have the greatest biological activity on carotenoid accumulation. At 259 DAT, fruits harvested from 50 ppm MBTA-treated trees showed a 68% increase in carotenoid content, a 10% increase in brix, and an 8% increase in juice recovery when compared with the values of controls.

**TABLE 7**

| Enhanced juice recovery and reduced peel thickness of Valencia sweet orange by tertiary-amine bioregulators | | | |
|---|---|---|---|
| | DAYS AFTER BIOREGULATOR TREATMENT | | |
| | 181 | 215 | 259 |
| Fruit Size (mm) | 65.1 ± 0.3 | 64.8 ± 0.7 | 67.1 ± 0.6 |

| Juice Recovery (ml juice/fresh wt) | | | |
|---|---|---|---|
| Control | 0.48a | 0.47b | 0.51b |
| DCPTA-50 | 0.49a | 0.53a | 0.53ab |
| DCPTA-100 | 0.48a | 0.49b | 0.57a |
| DCBTA-50 | 0.48a | 0.50ab | 0.53ab |
| DCBTA-100 | 0.49a | 0.50ab | 0.53ab |
| MBTA-50 | 0.49a | 0.50ab | 0.55a |
| MBTA-100 | 0.47a | 0.51a | 0.55a |
| MBTA-200 | 0.46a | 0.52a | 0.54ab |

| Peel Thickness (mm) | | | |
|---|---|---|---|
| Control | 4.8b | 5.0c | 4.9c |
| DCPTA-50 | 4.3a | 4.4b | 4.5bc |
| DCPTA-100 | 4.4ab | 4.5b | 4.2b |
| DCBTA-50 | 4.5ab | 4.4b | 4.4b |
| DCBTA-100 | 4.6b | 4.5b | 4.4bc |
| MBTA-50 | 4.2a | 4.0a | 3.8a |
| MBTA-100 | 4.5ab | 4.4b | 4.2b |
| MBTA-200 | 4.7b | 4.4b | 4.3b |
| Letters within columns indicate mean separations according to Duncan's multiple range test, 5% level. | | | |

**TABLE 8**

| Enhanced pigment accumulation of Valencia sweet orange by tertiary-amine bioregulators | | | | |
|---|---|---|---|---|
| | | DAYS AFTER BIOREGULATOR TREATMENT | | |
| | | 181 | 215 | 259 |
| Total Chlorophylls | Control | 0.60c | 0.47c | 0.41b |
| | DCPTA-50 | 0.27b | 0.19b | 0.17ab |
| | DCPTA-100 | 0.21ab | 0.18b | 0.12a |
| | DCBTA-50 | 0.20ab | 0.19b | 0.14a |
| | DCBTA-100 | 0.16a | 0.15ab | 0.12a |
| | MBTA-50 | 0.23b | 0.14ab | trace |
| | MBTA-100 | 0.10a | 0.12a | 0.12a |
| | MBTA-200 | 0.22ab | 0.15ab | 0.11a |
| Total Carotenoids | Control | 2.07d | 2.99c | 3.46c |
| | DCPTA-50 | 2.35c | 4.00b | 4.31b |
| | DCPTA-100 | 2.28c | 4.27b | 4.83ab |
| | DCBTA-50 | 2.71ab | 5.01ab | 5.46a |
| | DCBTA-100 | 2.86ab | 4.87ab | 5.55a |
| | MBTA-50 | 3.05a | 5.43a | 5.82a |
| | MBTA-100 | 2.64b | 4.99ab | 5.77a |
| | MBTA-200 | 2.85ab | 4.92ab | 5.41a |
| Letters within columns indicate mean separations according to Duncan's multiple range test, 5% level. | | | | |

### EXAMPLE 6

At Republic Groves of Hardee County, Florida five year-old trees of Hamlin Sweet Orange were treated with the following individual compounds and mixtures in the following concentrations:
DCBTA 10, 50, 100 ppm
MBTA 10, 50, 100 ppm

MBTA/DCBTA mixture 1, 10, 50 ppm of each compound All bioregulator solutions contained 0.1% Tween 80™ (w/v). All trees received a single foliage spray application of chemical as a complete canopy spray. Fruit sizes ranged from 9 to 12 mm in diameter at time of chemical treatment. Control trees received a single application of 0.1% Tween 80™. Each treatment consisted of five trees. Mature fruits were harvested from each treatment at 6 months after chemical treatment.

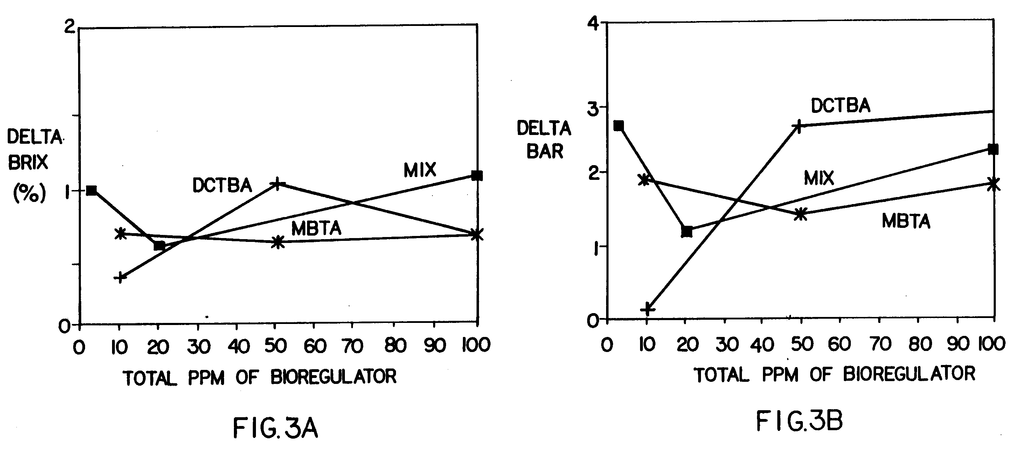

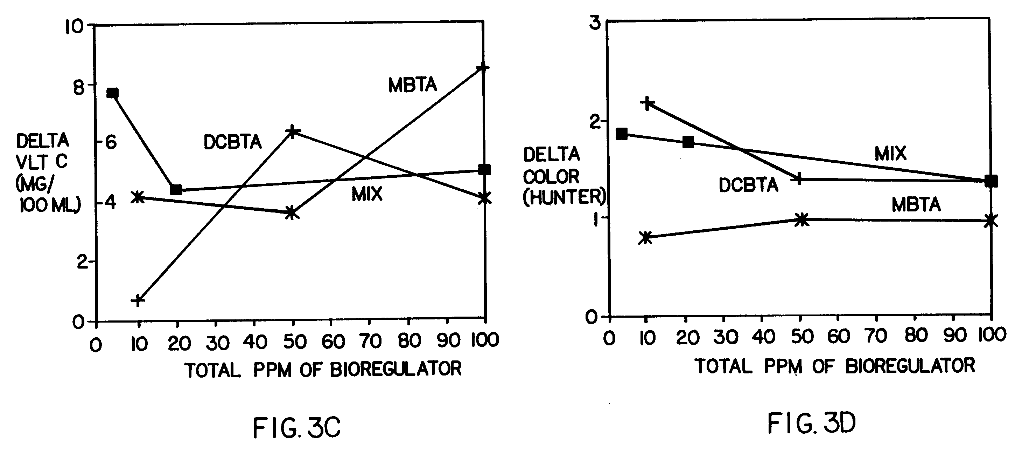

Application of a mixture of MBTA and DCBTA to Hamlin sweet orange trees at the beginning of fruit growth significantly enhanced the juice content (Table 9) and juice quality (Figure 3 a-d) of mature fruits when compared with the values of controls. Among all treatments, chemical improvements of peel development and juice content (Table 9) were greatest within the MIX 10/10 treatment. Among the mixture treatments, the MIX 1/1 treatment showed the largest numerical improvements in juice quality (Figure 3). In general, the MIX 1/1 treatment showed the largest numerical improvements in juice brix, juice BAR, and juice vitamin C content, when the chemical treatments contained less than 50 ppm bioregulator. Mixture (MIX 1/1 and MIX 10/10) treatments showed improvements in juice color when compared with 10 ppm MBTA and 10 ppm DCBTA treatments. These results suggest that the biological activities of MIX treatments, when used in low concentrations, are increased relative to the bioactivity of a single chemical treatment. Thus, the use of a mixture of MBTA and DCBTA as foliage treatments allows less chemical to be applied per tree, when compared with single chemical treatments.

**Table 9**

| **Promotive effects of tertiary amine bioregulators on fruit development of Hamlin sweet orange.** | | | | | | | |
|---|---|---|---|---|---|---|---|
| Treatment | | Fruit Diameter (mm) | Peel Thickness (mm) | Fruit Composition(%) | | | TOTAL |
| | | | | Juice | Peel | Pulp+ Seeds | |
| CONTROL | | 67.6 | 3.4 | 51.5 | 38.1 | 7.6 | 97.2 |
| DCBTA | -10 | 67.6 | 3.0 | 53.9 | 36.2 | 8.0 | 98.1 |
| | -50 | 68.4 | 2.8 | 54.0 | 35.5 | 8.6 | 98.1 |
| | -100 | 65.8 | 3.3 | 53.4 | 37.2 | 7.6 | 98.2 |
| MBTA | -10 | 66.6 | 3.0 | 54.2 | 35.9 | 8.1 | 98.2 |
| | -50 | 67.8 | 2.9 | 54.1 | 35.8 | 8.7 | 98.6 |
| | -100 | 66.1 | 3.0 | 54.4 | 35.5 | 9.3 | 99.2 |
| Mix² | 1/1 | 69.4 | 2.8 | 53.1 | 34.9 | 11.3 | 99.3 |
| | 10/10 | 69.2 | 2.2 | 57.9 | 30.5 | 9.9 | 98.3 |
| | 50/50 | 68.4 | 3.1 | 56.8 | 33.5 | 9.2 | 99.5 |

| | | | | | | | |
|---|---|---|---|---|---|---|---|
| ²Mixture of DCBTA & MBTA, (1/1,w/v) | | | | | | | |

### EXAMPLE 7

The propagation of epiphytic orchids requires plant cultivation in vitro. Arditti, J. 1977. Clonal propagation of Orchids by Means of Tissue Culture-A Manual. In: J. Arditti, (ed), Orchid Biology, Reviews and Perspectives, pp. 203-293. Cornell Univ. Press, Ithaca, New York. Orchid seeds and meristem propagations are cultured on aseptic, artificial media for up to 3 years before the seedling plants are grown for 3 to 5 years to produce mature, blooming plants.

Compared with controls, application of DCPTA to seedling phalaenopsis orchids during routine seedling transfer from aseptic growth in vitro to greenhouse-culture significantly enhanced plant growth and reduced the time to flowering. In addition, seedling death after transplanting was reduced significantly within the DCPTA treatment groups as compared with controls.

In this example, the growth promoting effects of MBTA, DCBTA, DCPTA, and a mixture of MBTA/DCBTA on seed germination and protocorm development in vitro of Brassolaeliocattleya orchid are examined. Protocorm (germinated seed) development represents the initial growth stage of orchid plant development in vitro. Leaf and root meristems differentiate from the unspecialized protocorm cells to produce a functional orchid seedling.

All orchid seeds were sown on sterilized Hill's seed germination medium obtained from Gallup and Stribling Laboratories, Santa Barbara, CA. The solid-support medium contained a mixture of buffered mineral salts, auxin, cytokinin, amino acids, organic acids, and agar. The medium (34 g/liter) was solubilized in distilled water and was adjusted to a Ph of 5. All orchid cultures were grown in 65 X 65 X 100 mm plastic vessels (Magenta Corporation, Chicago, IL) that contained 100 ml seed germination medium. All culture vessels were autoclaved for 10 min at 1,03 * 10⁵ (15 psi). After medium sterilization 10 ppb MBTA, 10 ppb DCBTA, 10 ppb DCPTA, and a 10 + 10 ppb mixture of MBTA and DCBTA were filter-sterilized and were added to the medium (5 ml/vessel) before the agar-support solidified. The bioregulator solutions were prepared in distilled water. Experimental controls received 5 ml aliquots of filter-sterilized water. All treatment groups contained three replicate vessels.

All orchid seed transfers were performed under sterile conditions. Dry seeds of Brassolaeliocattleya X Ruben's Verde (Blc. Green Heart 'Imperial Jade' x Blc. Lester McDonald 'Kelly' AM/AOS) were surface sterilized for 20 minutes. The seeds were then sown, without rinsing, on the bioregulator-amended media. Seeds were distributed evenly on the medium surface with gentle shaking. Seeds were germinated at 23°C under continuous illumination (75 µE m⁻² s⁻¹) using two wide-spectrum fluorescent lamps.

Days to seed germination were recorded for each bioregulator treatment. Sixty days after seed sowing, protocorms were harvested from each vessel and the total protocorm fresh weight was determined. Fresh weight and diameters for 50 protocorms were determined. Each group of 50 protocorms was extracted into 100% acetone and the chlorophyll and total carotenoids contents were quantified.

Orchid seed germination and protocorm development was enhanced significantly by the addition of tertiary amine bioregulators to the aseptic culture medium (Table 10). During seed sowing, visually equal amounts of seeds were plated into each vessel. Seeds were observed to germinate 13 days after sowing among the control, DCPTA- amended, and DCBTA-amended cultures. However, seeds plated on the MBTA/DCBTA mixture and MBTA-amended cultures were observed to germinate 10 days after seed sowing. Compared with controls, protocorm fresh weight was increased significantly (P = 0.05) by the addition of bioregulators to the in vitro culture medium (Table 11). Chlorophyll a, chlorophyll b, and total carotenoid contents of mature protocorms grown on tertiary amine bioregulator-amended media (TAB-media) were increased significantly compared with controls. However, the chlorophyll a to b ratios of all treatment groups were statistically similar. Of the compounds that were tested, the MBTA and MBTA/DCBTA mixture treatments showed the largest numerical improvements in protocorm fresh weight and pigment accumulation when compared with controls.

This study indicates that orchid protocorm growth in vitro is enhanced significantly by the addition of tertiary amine bioregulators to an aseptic culture medium (Table 10). Among the compounds that were tested, treatments that contained MBTA showed the greatest improvements in seed germination, protocorm growth, and total pigment accumulation when compared with controls. The chlorophyll a to b ratios of control and all chemical treatments were numerically similar. These results suggest that chloroplast size (volume) or chloroplast number per cell was enhanced in response to tertiary amine-treatment, rather than a specific enhancement of chlorophyll a or chlorophyll b biosynthesis. The potentially enhanced chloroplast size of orchid protocorms grown in TAB-media is significant, since the total chloroplast volume per cell generally determines the photosynthetic carbon fixation rate and cell growth rate of plants.

**Table 10**

| Treatment^{z} | Protocorm fresh wt (g x 50) | Pigment content | | (µg/g fresh wt) | |
|---|---|---|---|---|---|
| | | Chla | Chlb | Chla/b | Cartot |
| Control | 0.27 d | 57.3 c | 31.7 b | 1.81 a | 32.1 b |
| DCPTA-10 ppb | 0.38 b | 62.5 b | 34.8 ab | 1.80 a | 36.4 ab |
| DCBTA-10 ppb | 0.35 c | 66.1 ab | 35.5 ab | 1.86 a | 38.5 a |
| MBTA-10 ppb | 0.39 b | 69.3 a | 37.8 a | 1.83 a | 41.0 a |
| MBTA/DCBTA-10 +10 ppb | 0.43 a | 69.7 a | 38.1 a | 1.83 a | 40.7 a |

| | | | | | |
|---|---|---|---|---|---|
| ^{z} DCPTA, N,N-diethylaminoethyl 3,4-dichlorophenylether DCBTA, N,N-diethylaminoethyl 3,4-dichlorobenzylether MBTA, N,N-diethylaminoethyl 4-methylbenzylether | | | | | |

## Claims

1. Method for enhancing plant growth comprised of the step of applying to a plant a compound of the Formula I 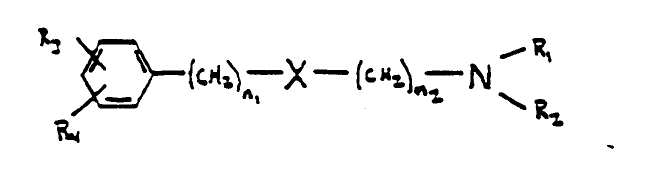 wherein
X is either oxygen or sulphur,
R₁ and R₂ are lower alkyl containing 1 to 6 carbon atoms, n₁ and n₂ being integers from 1 to 6 each, n₁ and n₂ being independent of each other;
R₃ and R₄ are independently hydrogen, chlorine, fluorine, bromine, iodine, lower alkyl containing 1 to 6 carbon atoms, lower alkoxy compounds containing 1 to 6 carbon atoms,
and
wherein:
if R₃ and R₄ are 3,5-substituents, then the lower alkyl or alkoxy group must contain 3 to 6 carbon atoms; and wherein: if R₃ is hydrogen, then R₄ must be a 4-substituent, with the proviso that R₄ is other than hydrogen;
or an acid salt thereof.

2. Method according to claim 1, **characterized** in that a mixture of at least two of the compounds of the Formula 1 is applied.

3. Method according to claim 2, **characterized** in that the applied mixture shows a synergistic growth enhancing effect.

4. Method for enhancing plant growth according to any of the claims 1 to 3, **characterized** by applying the compound to the plant immediately prior to or at a time when cell differentiation and growth of the plant or flower buds are great, that is, to seeds, to plant seedlings, or to trees during flower bud initiation, bud swell, or during a period of exponential vegetative growth, said compounds being applied in an amount enhancing plant growth but not harming the plant and being applied in an amount of 0,001 to 0,3 mg active ingredient per plant seedling or 0.01 to up to 10 mg active ingredient per tree, said enhancing of the plant growth consisting of an increase in photosynthesis, total plant biomass and plant constituents selected from the group consisting of protein, lipid, sugar, and essential oil.

5. Method according to any of the claims 1 to 4, wherein the plant is propagated in vitro.

6. Method according to any of the claims 1 to 5, wherein the enhancing of plant growth further consists of an increased pigment accumulation, total soluble solids, vitamin, nutrient, or juice contents of fruit harvested from treated plants relative to untreated controls.

7. Method according to any of the preceding claims, wherein the compound is applied as an aqueous solution.

8. Method according to any of the preceding claims, wherein the compound is applied to the plant more than once.

9. Method according to any of the preceding claims, wherein the enhancing of plant growth further consists of an accelerated structural maturation of the plant and reduction of days to crop harvest relative to untreated plants.

10. Method according to any of the preceding claims, wherein the plant is derived from an annual or perennial agronomic crop plant, a woody plant tissue, or from a plant grown for ornamental purposes.

11. Method according to claim 10, wherein the plant is a cereal grain.

12. Method according to claim 10, wherein the plant is a legume.

13. Method according to claim 10, wherein the plant is a citrus tree.

14. Method according to claim 13, wherein the fruit of the treated citrus tree exhibits a reduced peel thickness relative to untreated citrus.

15. Method according to claim 10, wherein the plant is a vegetable plant.

16. Compound of the Formula I capable of enhancing plant growth 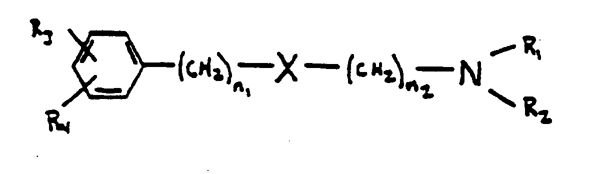 wherein n₁ is 1 and n₂ is 2, X is oxygen, R₁ and R₂ are both ethyl and the benzyl ring in Formula I substituted by R₃ and R₄ is chosen from the group consisting of 2,4-dichlorobenzyl; 3,4-dichlorobenzyl; 3,5-diisopropylbenzyl; 3,5-ditertiary butylbenzyl; 3,4-dimethylbenzyl; 3,4-dimethoxybenzyl; 3-methylbenzyl, 4-methylbenzyl and 4-chlorobenzyl and acid addition salts thereof.

17. Compounds of the Formula I capable of enhancing plant growth according to claim 16, wherein the compound is N,N-diethylaminoethyl dichlorobenzylether or an acid salt thereof.

18. Compounds of the Formula I capable of enhancing plant growth according to claim 16, wherein the compound is N,N-diethylaminoethyl-4-methylbenzylether or an acid salt thereof.

19. A mixture of at least two of the compounds set forth in claims 16, 17 or 18.

20. A mixture according to claim 19, wherein the mixture shows a synergistic growth enhancing effect.

## Patentansprüche

1. Verfahren zur Förderung des Pflanzenwachstums, umfassend den Schritt der Verabreichung an eine Pflanze einer Verbindung der Formel I 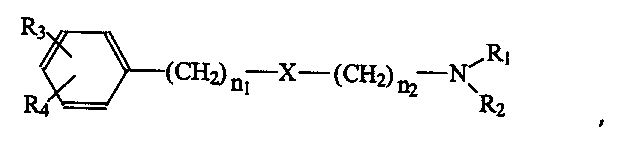 in der
X entweder ein Sauerstoff- oder Schwefelatom ist,
R₁ und R₂ Niederalkylreste mit 1 bis 6 Kohlenstoffatomen sind, n₁ und n₂ jeweils ganze Zahlen mit einem Wert von 1 bis 6 sind, n₁ und n₂ voneinander unabhängig sind;
R₃ und R₄ unabhängig voneinander Wasserstoff-, Chlor-, Fluor-, Brom-, Iodatome, Niederalkylreste mit 1 bis 6 Kohlenstoffatomen, Niederalkoxyverbindungen mit 1 bis 6 Kohlenstoffatomen sind,
und in der
der Niederalkyl- oder Niederalkoxyrest 3 bis 6 Kohlenstoffatome enthält, wenn R₃ und R₄ 3,5-Substituenten sind; und R₄ ein 4-Substituent ist, wenn R₃ ein Wasserstoffatom ist, mit der Maßgabe, daß R₄ kein Wasserstoffatom ist;
oder ein saures Salz davon.

2. Verfahren nach Anspruch 1, dadurch gekennzeichnet, daß ein Gemisch von mindestens zwei der Verbindungen der Formel I verabreicht wird.

3. Verfahren nach Anspruch 2, dadurch gekennzeichnet, daß das verabreichte Gemisch eine synergistische wachstumsfördernde Wirkung zeigt.

4. Verfahren zur Förderung des Pflanzenwachstums nach einem der Ansprüche 1 bis 3, dadurch gekennzeichnet, daß die Verbindung an die Pflanze unmittelbar vor oder zu einem Zeitpunkt verabreicht wird, an dem die Zelldifferenzierung und das Wachstum der Pflanze oder der Blütenknospen bedeutend sind, d.h. an Samen, Pflanzensämlinge oder Bäume während der Initiation der Blütenknospung, der Knospenschwellung oder während eines Zeitraums von exponentiellem vegetativem Wachstum, wobei die Verbindungen in einer Menge, die das Pflanzenwachstum fördern, jedoch die Pflanze nicht schädigen, von 0,001 bis 0,3 mg Wirkstoff pro Pflanzensämling oder 0,01 bis 10 mg Wirkstoff pro Baum verabreicht werden und wobei die Förderung des Pflanzenwachstums aus einer Zunahme der Photosynthese, der Gesamtbiomasse der Pflanze und der Bestandteile der Pflanze, ausgewählt aus Protein, Lipid, Zucker und essentiellem Öl, besteht.

5. Verfahren nach einem der Ansprüche 1 bis 4, wobei die Pflanze in vitro vermehrt wird.

6. Verfahren nach einem der Ansprüche 1 bis 5, wobei die Förderung des Pflanzenwachstums weiter aus einer erhöhten Pigmentanhäufung, einem erhöhten Gesamtgehalt an löslichen Feststoffen, Vitaminen, Nährstoffen oder Saft bei den Früchten, die von behandelten Pflanzen geerntet werden, im Vergleich zu unbehandelten Kontrollen besteht.

7. Verfahren nach einem der vorhergehenden Ansprüche, wobei die Verbindung als eine wäßrige Lösung verabreicht wird.

8. Verfahren nach einem der vorhergehenden Ansprüche, wobei die Verbindung an die Pflanze mehr als einmal verabreicht wird.

9. Verfahren nach einem der vorhergehenden Ansprüche, wobei die Förderung des Pflanzenwachstums weiter aus einer beschleunigten strukturellen Reifung der Pflanze und Verminderung der Tage bis zum Ernten der Feldfrüchte im Vergleich zu unbehandelten Pflanzen besteht.

10. Verfahren nach einem der vorhergehenden Ansprüche, wobei die Pflanze von einer einjährigen oder mehrjährigen landwirtschaftlich genutzten Feldfruchtpflanze, einem hölzernen Pflanzengewebe oder von einer Pflanze, die als Zierpflanze gezüchtet wird, abstammt.

11. Verfahren nach Anspruch 10, wobei die Pflanze Getreide ist.

12. Verfahren nach Anspruch 10, wobei die Pflanze ein Hülsenfrüchtler ist.

13. Verfahren nach Anspruch 10, wobei die Pflanze ein Citrusbaum ist.

14. Verfahren nach Anspruch 13, wobei die Frucht des behandelten Citrusbaumes eine verminderte Schalendicke im Vergleich zur unbehandelten Citrusfrucht aufweist.

15. Verfahren nach Anspruch 10, wobei die Pflanze eine Gemüsepflanze ist.

16. Verbindung der Formel I, die das Pflanzenwachstum fördern kann, 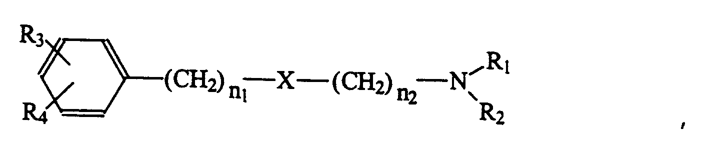 in der n₁ den Wert 1 und n₂ den Wert 2 aufweisen, X ein Sauerstoffatom ist, R₁ und R₂ beide Ethylgruppen sind und der Benzylring der Formel I, der mit R₃ und R₄ substituiert ist, eine 2,4-Dichlorbenzyl-, 3,4-Dichlorbenzyl-, 3,5-Diisopropylbenzyl-, 3,5-Di-tert-butylbenzyl-, 3,4-Dimethylbenzyl-, 3,4-Dimethoxybenzyl-, 3-Methylbenzyl-, 4-Methylbenzyl- oder 4-Chlorbenzylgruppe, ist und Säureadditionssalze davon.

17. Verbindungen der Formel I, die zur Förderung des Pflanzenwachstums fähig sind, nach Anspruch 16, wobei die Verbindung N,N-Diethylaminoethyldichlorbenzylether oder ein saures Salz davon ist.

18. Verbindungen der Formel I, die zur Förderung des Pflanzenwachstums fähig sind, nach Anspruch 16, wobei die Verbindung N,N-Diethylaminoethyl-4-methylbenzylether oder ein saures Salz davon ist.

19. Gemisch von mindestens zwei der in einem der Ansprüche 16, 17 oder 18 dargestellten Verbindungen.

20. Gemisch nach Anspruch 19, wobei das Gemisch eine synergistische wachstumsfördernde Wirkung zeigt.

## Revendications

1. Procédé pour améliorer la croissance des plantes comprenant l'étape consistant à appliquer sur une plante un composé répondant à la formule I 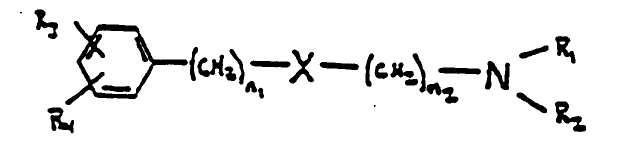 dans laquelle
X est l'atome d'oxygène ou de soufre,
R₁ et R₂ représentent des groupes alcoyles inférieurs de 1 à 6 atomes de carbone, n₁ et n₂ sont des nombres entiers tous deux compris entre 1 et 6, n₁ et n₂ étant indépendants l'un de l'autre ;
R₃ et R₄ représentent indépendamment l'atome d'hydrogène, de chlore, de fluor, de brome ou d'iode, un groupe alcoyle inférieur de 1 à 6 atomes de carbone,des groupes alkoxy inférieurs de 1 à 6 atomes de carbone,
et dans laquelle:
si R₃ et R₄ sont des substituants en 3,5-, le groupe alcoyle inférieur ou le groupe alkoxy inférieur doit comporter de 3 à 6 atomes de carbone ;
et dans laquelle:
si R₃ représente l'atome d'hydrogène, alors R₄ doit être un substituant 4-, avec la condition que R₄ soit différent de l'atome d'hydrogène ;
ou un sel acide de celui-ci.

2. Procédé selon la revendication 1, caractérisé en ce qu'un mélange d'au moins deux des composés répondant à la formule I est appliqué.

3. Procédé selon la revendication 2, caractérisé en ce que le mélange appliqué exerce un effet synergique d'amélioration de la croissance.

4. Procédé pour améliorer la croissance des plantes selon l'une quelconque des revendications 1 à 3, caractérisé en ce qu'il comprend l'application du composé sur la plante immédiatement avant ou au moment où la différenciation cellulaire et la croissance de la plante ou des boutons floraux sont grandes, en d'autres termes, aux semences, aux jeunes plants, ou aux arbres lors de l'apparition des boutons floraux, de la croissance des bourgeons, ou durant une période de croissance végétative exponentielle, lesdits composés étant appliqués dans une quantité améliorant la croissance de la plante mais ne nuisant pas à la plante, lesdits composés étant appliqués dans une quantité de 0,001 à 0,3 mg d'ingrédient actif par jeune plant ou de 0,01 jusqu'à 10 mg d'ingrédient actif par arbre, ladite amélioration de la croissance végétale consistant en une augmentation de la photosynthèse, de la biomasse végétale totale et des constituants de la plante choisis parmi le groupe consistant en protéines, lipides, sucres et huiles essentielles.

5. Procédé selon l'une quelconque des revendications 1 à 4, dans lequel la plante fait l'objet d'une propagation *in vitro*.

6. Procédé selon l'une quelconque des revendications 1 à 5, dans lequel l'amélioration de la croissance des plantes consiste en outre en un accroissement de l'accumulation de pigments, de la teneur en solides solubles totaux, en vitamines, en nutriments ou en jus des fruits récoltés à partir de plantes traitées par comparaison avec des témoins non traités.

7. Procédé selon l'une quelconque des revendications précédentes, dans lequel le composé est appliqué sous la forme d'une solution aqueuse.

8. Procédé selon l'une quelconque des revendications précédentes, dans lequel le composé est appliqué sur la plante plus d'une fois.

9. Procédé selon l'une quelconque des revendications précédentes, dans lequel l'amélioration de la croissance des plantes consiste en outre en une accélération de la maturation structurelle de la plante et en une réduction du nombre de jours avant récolte par comparaison avec les plantes non traitées.

10. Procédé selon l'une quelconque des revendications précédentes, dans lequel la plante provient d'un plante annuelle ou pérenne cultivée à des fins agronomiques, d'un tissu végétal ligneux, ou d'une plante cultivée à des fins ornementales.

11. Procédé selon la revendication 10, dans lequel la plante est un grain de céréale.

12. Procédé selon la revendication 10, dans lequel la plante est une légumineuse.

13. Procédé selon la revendication 10, dans lequel la plante est un agrume.

14. Procédé selon la revendication 13, dans lequel le fruit de l'agrume traité présente une écorce d'épaisseur réduite par comparaison avec l'agrume non traité.

15. Procédé selon la revendication 10, dans lequel la plante est un légume.

16. Composé répondant à la formule I capable d'améliorer la croissance des plantes 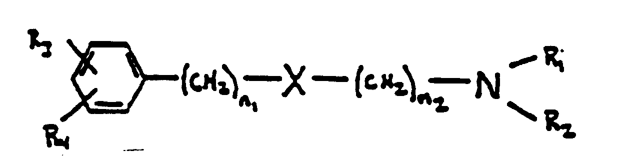 dans laquelle n₁ est égal à 1 et n₂ est égal à 2, X représente l'atome d'oxygène, R₁ et R₂ représentent tous deux le groupe éthyle, et le cycle benzyle dans la formule I substitué par R₃ et R₄ est choisi parmi le groupe consistant en 2,4-dichlorobenzyle ; 3,4-dichlorobenzyle ; 3,5-di-isopropylbenzyle ; 3,5-dibutylbenzyle tertiaire ; 3,4-diméthylbenzyle ; 3,4-diméthoxybezyle ; 3-méthylbenzyle ; 4-méthylbenzyle ; et 4-chlorobenzyle ; et les sels d'addition d'acide de ceux-ci.

17. Composés répondant à la formule I capables d'améliorer la croissance des plantes selon la revendication 16, dans lesquels le composé est le N,N-diéthylaminoéthyle dichlorobenzyle éther ou un sel acide de celui-ci.

18. Composés répondant à la formule I capables d'améliorer la croissance des plantes selon la revendication 16, dans lesquels le composé est le N,N-diéthylaminoéthyle 4-méthylbenzyle éther ou un sel acide de celui-ci.

19. Mélange d'au moins deux des composés revendiqués dans les revendications 16, 17 ou 18.

20. Mélange selon la revendication 19, dans lequel le mélange exerce un effet synergique d'amélioration de la croissance.
